# EUROPEAN PATENT SPECIFICATION

(11) **EP 0 549 974 B1**
(45) Date of publication and mention of the grant of the patent: **23.07.1997**
(21) Application number: 92121557.0
(22) Date of filing: 18.12.1992
(51) Int. Cl.: G01N 33/574

(54) **Immunoassay for NCA-BT in blood samples of breast cancer patients**
Immunoassay für NCA-BT in Blutproben von Brustkrebspatientinnen
Essai immunologique de NCA-BT dans le sang de patients avec cancer du sein

(30) Priority: 30.12.1991 US 815934
(43) Date of publication of application: 07.07.1993
(73) Proprietor: Bayer Corporation, Pittsburgh, PA 15219-2502 (US)
(72) Inventor: Elting, James J., Madison, CT 06443 (US); Barnett, Thomas R., Prospect, CT 06516 (US)
(74) Representative: Dänner, Klaus, Dr.

(56) References cited:
- EP-A- 0 092 223
- EP-A- 0 446 602
- BIOCHEMICAL AND BIOPHYSICAL RESEARCH COMMUNICATIONS vol. 150, no. 1, 15 January 1988, NEW YORK, NY. pages 89 - 96 TAWARAGI ET AL. 'Primary structure of nonspecific crossreacting antigen (NCA), ........'
- ONCODEVELOPMENTAL BIOLOGY AND MEDICINE, vol. 4, 1983, AMSTERDAM, pp. 209-217, CHAVANEL ET AL. 'Production of monoclonal antibodies against the nonspecific cross-reacting antigen (NCA)'
- ACTA PATHOLOGICA JAPONICA vol. 40, no. 2, 1990, TOKYO pages 85 - 97 TSUTSUMI ET AL. 'Immunohistochemical demonstration of nonspecific cross-reacting antigen in normal and neoplastic ....'
- THE JOURNAL OF IMMUNOLOGY vol. 137, no. 3, 1 August 1986, BALTIMORE, MD. pages 839 - 845 BURTIN ET AL. 'Antigenic variants of the nonspecific cross-reacting antigen (NCA)'

## Description

### BACKGROUND OF THE INVENTION

The present invention relates to monitoring the progression or stage of disease in breast cancer patients. More particularly, the invention relates to such monitoring methods based on measurement of cancer marker blood levels.

A number of substances have been determined to be useful markers for use in monitoring the course of various cancer diseases. Some useful markers that have been identified are onofetal antigens such as carcinoembryonic antigen (CEA) and alpha-fetoprotein, tissue-specific antigens such as prostate-specific antigen (PSA), and mucin antigens such as those conventionally known as CA-125 and CA-19-9. Immunoassays for antigens such as these are typically used as confirmatory tests at the time of diagnosis and subsequently for monitoring patient status. Occasionally, the use of such tests crosses the boundaries of tumor type (for example, the use of CEA tests in colon, breast, and lung cancer, and alpha-fetoprotein in hepato-cellular and testicular cancer), but the utility of each test type is foremost for a single tumor type (for example, PSA for prostate cancer and CA-125 for ovarian cancer).

A family of antigenic proteins have been identified which are genetically and immunologically related to CEA (Thompson, J. and W. Zimmerman (1988) Tumor Biol. 9, 63-83; and Barnett, T. and W. Zimmerman (1990) Tumor Biol. 11, 59-63). Among these are the non-specific cross-reacting antigens (NCAs), and the trans-membrane antigens (TM-CEAs). It has further been found that at least two species of NCA exist (Tawargi, Y., Oikawa, S., Matsuoka, Y., Kosaki, G. and H. Nakazato (1988) Biochem. Biophys. Res. Commun. 150, 89-96; and Neumaier, M., Zimmerman, W., Shively, L., Hinoda, Y., Riggs, A.D., and J.E. Shively (1988) J. Biol. Chem. 263, 3202-3207), and have been designated as NCA-BT and NCA-CML (with respect to NCA-BT, see Barnett, T., Goebel, S.J., Nothdurft, M.A., and J.J. Elting (1988) Genomics 3:59-66; Tawargi, Y., et al, supra; and Neumaier, M., Zimmerman, W., Shively, L., Hinoda, Y., Riggs, A., and J.E. Shively (1988) J. Biol. Chem. 263:3202-3207; and with respect to NCA-CML, see Arakawa, F., Kuroki, M., Misumi, Y,. Oikawa, S., Nakazato, H., and Y. Matsuoka (1990) Biochem. Biophys. Res. Commun. 166:1063-1071).

From a better understanding of the complex group of glycoproteins belonging to the carcinoembryonic antigen family has followed the observation that various members of this family are differentially expressed by various tumor types. Most well known is the fact that CEA itself is expressed by most if not all colorectal carcinomas while only by a minority of breast carcinomas. Because of the successful production of monoclonal antibodies specific to various members of this antigen family, it is now possible to screen sera from various cancer populations and to determine the incidence of elevation of individual members of the CEA family in various types of cancer. Prior to the generation of such specific antibodies, attempts to quantitate NCA levels in cancer sera were confounded by the presence of varying amounts of other members of the CEA family that cross-reacted with the immunoassay being used.

Although there have been reports of the successful generation of monoclonal antibodies specific for NCA (Chavanel, G., Frenoy, N., Escribano, M.J., and P. Burtin (1983) Oncodev. Biol. and Med. 4, 209-217), there have been no reports of a correlation between blood NCA levels and the clinical status of any particular cancer patients. There have also been reports on studies showing the presence of NCA in various tumor tissues (Acta Pathologica Japanica (1990) Vol. 40 (2), pages 85-97), however, as above these studies did not investigate any relationship between blood levels and disease state.

In fact, there are specific statements in the literature that NCA is a very poor marker for cancer (Shively, J.E., Spayth, V., Chang, F.-F., Metter, G.E., Klein, L., Presant, C.A., and C.W. Todd (1982) Cancer Res. 42:2502-2513; and Burtin, P., Chavanol, G., Hendrick, J.C., and N. Frenoy, (1986) J. Immunol. 137:839-845). It has been further speculated that immunoassays based on monoclonal antibodies such as are now widely accepted for CEA and other antigens would be much more difficult to develop (Burtin, P. et al, supra).

### SUMMARY OF THE INVENTION

It has now been found that changes in the blood level of NCA-BT in breast cancer patients provides a means for monitoring the progression of the disease. Increases in blood NCA-BT levels measured by performing a series of specific immunoassays over time indicate a deteriorating condition in a significant number of patients while decreases in blood NCA-BT levels indicate an improving condition in such patients.

### BRIEF DESCRIPTION OF THE DRAWINGS

Fig. 1 is a graph showing the specificity of the particular immunoassay described in the Examples below for NCA-BT compared to CEA and TM-CEA.

Fig. 2 is a graph showing the specificity of the particular immunoassay described in the Examples below for NCA-BT compared to NCA obtained from the spleen and NCA-CML.

Figs. 3-8 are graphs showing a comparison of the correlations of NCA-BT and CEA blood levels with the stage of breast cancer in patients under treatment.

### DESCRIPTION OF THE PREFERRED EMBODIMENTS

Essentially any method may be employed in the measurement of blood (e.g., serum or plasma) NCA-BT levels. Typically, such measurement will be performed by sandwich immunoassay using two antibody reagents, one of which is specific for NCA-BT (showing no substantial reactivity with other CEA family member antigens, particularly CEA, NCA-CML, and TM-CEA), while the other is capable of binding specifically or nonspecifically with NCA-BT. Assay format and methods for the preparation of the required antibody reagents can be selected by the skilled worker in the field. Suitable antibody reagents can be labeled, e.g., enzyme-labeled, or immobilized, e.g., coated onto a microtiter plate, bound to plastic or magnetic beads or particles, and can be comprised of whole immunoglobulins, e.g., IgG or IgM, or fragments, e.g., Fab, Fab', and F(ab')₂ fragments, or aggregates thereof.

Preferably, the NCA-BT specific antibody reagent is prepared by immunization of a host animal with a suitable immunogen such as an NCA-BT containing immunogen mixture, e.g., a purified extract of spleen or tumor cells; NCA-BT-expressing transfectant cell lines (see European Patent Publication 346,702); an immunogen conjugate comprising a synthetically prepared peptide coupled to a conventional immunogenic carrier molecule, where the peptide has an amino acid sequence encompassing an epitope of NCA-BT; and the like as will be understood in the art.

Antibody reagents comprising monoclonal antibodies will be generally preferred.

It will be understood that, similar to other types of accepted disease monitoring methods, the present method will not be useful on every patient diagnosed with breast cancer. Rather, the physician will use NCA-BT blood values in combination with other diagnostic values and clinical observations to deveiop a course of treatment and therapy for each individual patient. It is also contemplated that measurement of blood NCA-BT will provide a means for detecting the onset of breast cancer, thereby providing a method for screening a select patient population for the disease.

The present invention will now be illustrated, but is not intended to be limited by, the following examples.

### EXAMPLES

Mab 228.2 - BALB/C mice were immunized with 50 µg of an emulsion of NCA purified from human spleen (von Kleist, S. and P. Burtin (1969) Cancer Res. 29:1961-1964), and Freund's complete adjuvant. Spleens from hyperimmune animals were removed from euthanized animals and the splenocytes were fused with AG8 mouse myeloma cells (ATCC CRL 1580). The resulting hybridomas were screened for anti-NCA antibody production by sandwich ELISA. Positive clones were subsequently screened for anti-CEA and anti-TM-CEA(BGP) activity (see Barnett, T. and W. Zimmerman supra; and see Barnett, T.R., Kretschmer, A., Austen, D.A., Goebel, S.J., Hart, J.T., Elting, J.J., and M.E. Kamarck (1989) J. Cell Biol. 108:267-276) by sandwich ELISA assay. Those clones specific for NCA-BT were recloned and rechecked for cross reactivity with CEA and TM-CEA(BGP) by ELISA and again by FACS analysis using recombinant mouse cell lines expressing CEA, NCA-BT or TM-CEA on their plasma membranes (see European Patent Publication No. 346,702). The result of this screening process was identification of Mab 228.2 which is specific for NCA-BT with no detectable reactivity with CEA, NCA-CML or TM-CEA by ELISA.

Mab 176.7.5 - BALB/C mice were immunized with TM-CEA as described in EP-A 0 446 602.

The hybridoma that produces the selected antibody has been deposited with the American Type Culture Collection, Rockville, Maryland, USA, on April 5, 1990, and given the designation ATCC HB-10411. This antibody reacts with both NCA and TM-CEA but not with CEA. This antibody was crosslinked to calf intestine alkaline phosphatase (Biozyme Corp., San Diego, CA, USA) after thiolation of the antibody with 2-iminothiolane using the heterobifunctional cross-linking reagent sulfo-SMPB (Pierce Chem. Co., Rockford, IL, USA). This conjugate is hereafter designated 176.7.5-A.P.

NCA Calibrator - NCA purified from human spleen (von Kleist, S. et al, supra) was used as the calibrator for the NCA-BT assay. The amount of antigen was determined by amino acid analysis of a hydrolyzed sample of the purified NCA. Assay calibrators were made by diluting purified NCA into PBST-BSA (see below).

NCA-Specific Immunoassay - A sandwich ELISA was configured using NCA-specific Mab 228.2 as the solid phase capture antibody and Mab 176.7.5-A.P. as the reporter antibody. Mab 228.2 was coated in 100 µl of 0.1 M Na₂CO₃/NaHCO₃, pH 9.0, onto the wells of microtiter ELISA plates (product ID#25801, Corning Glass Works, Corning, NY, USA) for 1 hour at 37°C. The wells were blocked with 300 µl 0.1% Tween-20 (polyoxyethylenesorbitan monolaureate) in 0.05 M Na phosphate buffer, pH 7.2, 0.1 M NaCl and 0.01% Thimerosal (PBST) for 1 hour at 37°C. After washing the plate 5 times with PBST, 50 µl of antigen solution [calibrators in PBST with 5% bovine serum albumin (PBST-BSA) or serum unknowns] were added and incubated for 2 hours at 37°C. After washing 5 times with PBST, 100 µl of 176.7.5-A.P. (15 µg/ml in PBST) was added to each well and incubated for 1 hour at 37°C. After washing, 200 µl of p-nitrophenyl phosphate (in 1.0 M diethylamine buffer, pH 9.8) was added to each well and incubated for 30 minutes at room temperature. Absorbance at 405 nm was then determined and the amount of antigen in the test serum samples was determined from the calibrator standard curve.

Patient serum samples - Serum samples from clinically confirmed breast cancer patients were obtained from the University of Texas M.D. Anderson Cancer Center, Houston, TX, USA. Samples from clinically normal individuals were obtained from M.D. Anderson and from in-house volunteers.

### Results:

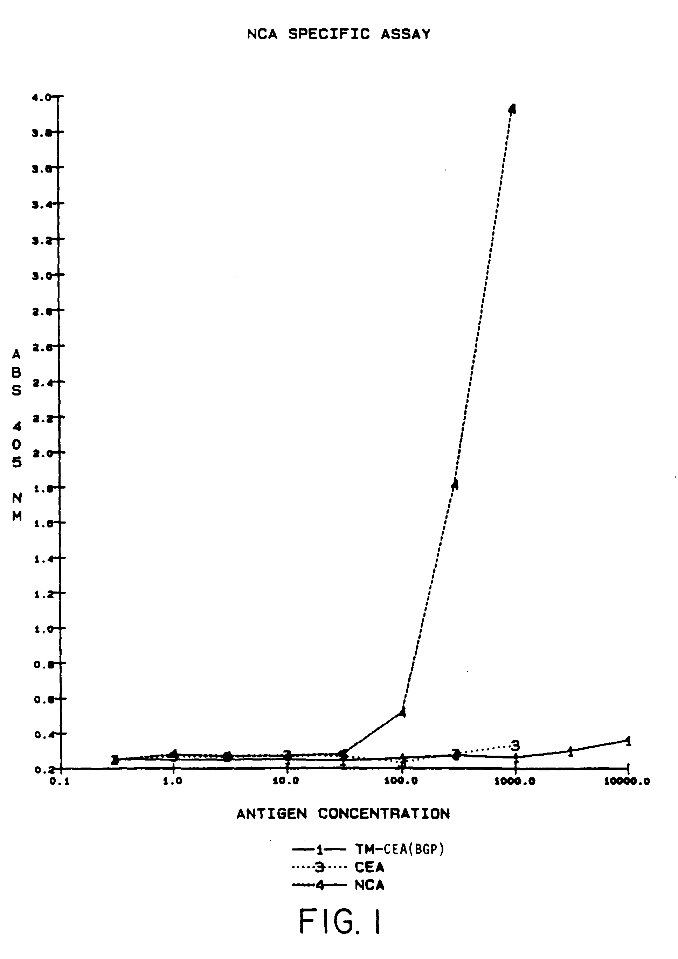

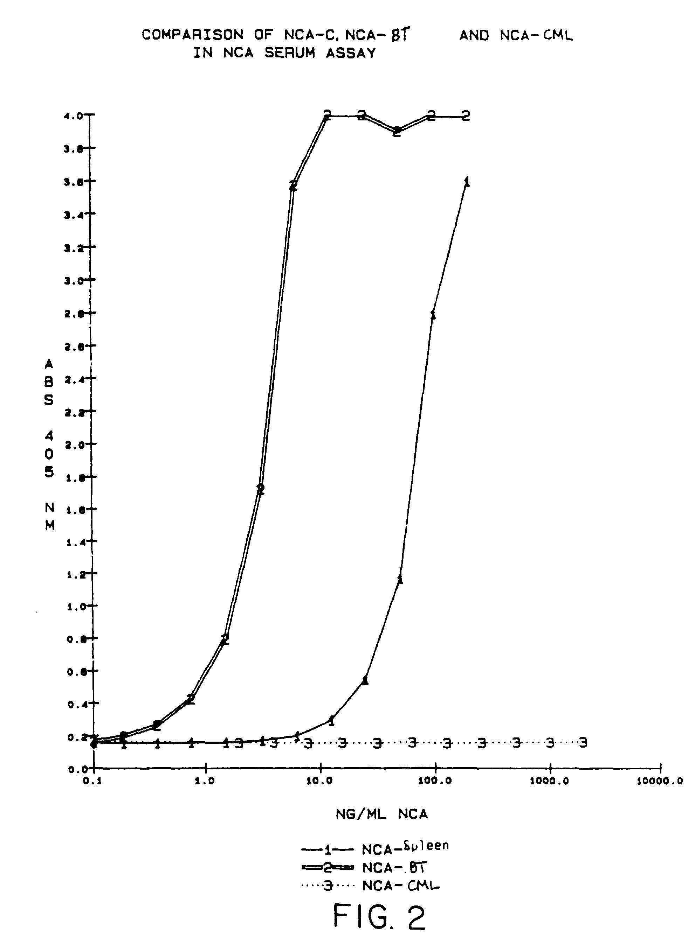

The antigenic specificity of the immunoassay for NCA-BT is established by the data shown in Figures 1 and 2. Figure 1 shows the lack of cross-reactivity with CEA and with TM-CEA(BGP). Testing with NCA-BT and NCA-CML purified from a recombinant expression vector containing cloned gene sequences (Figure 2) shows that the assay is specific for NCA-BT (NCA-spleen is NCA purified from human spleen as described in von Kleist, S. et al, supra).

NCA-BT levels in the serum of clinically normal females were determined and the cutoff (95th percentile confidence level) found to be 143 ng/ml (Table 1). The mean serum concentration of 202 clinically diagnosed breast cancer patients was 358 ng/ml.

**TABLE 1**

| Normals | Mean | 95th %ile |
|---|---|---|
| Female nonsmokers (n=50) | 64.9 ng/ml | 135 ng/ml |
| Female smokers (n=25) | 82.3 ng/ml | 161 ng/ml |
| Total Females (n=75) | 70.1 ng/ml | 143 ng/ml |

| Cancer Patients | | |
|---|---|---|
| Percent showing elevated NCA-BT | 358.1 ng/ml | 1756 ng/ml |
| levels = 66% | (p <0.0001) | |

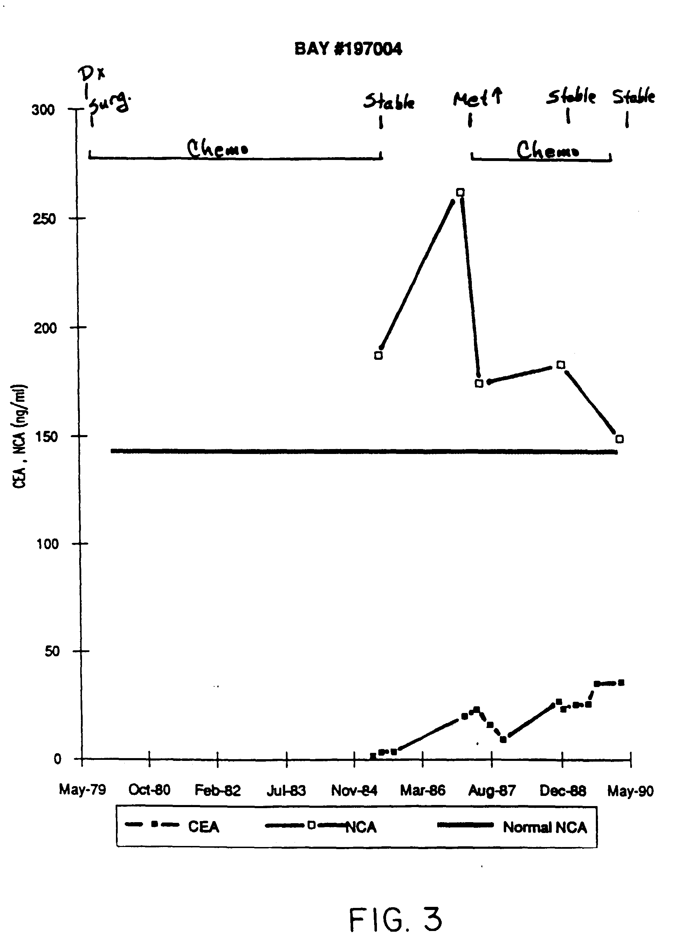

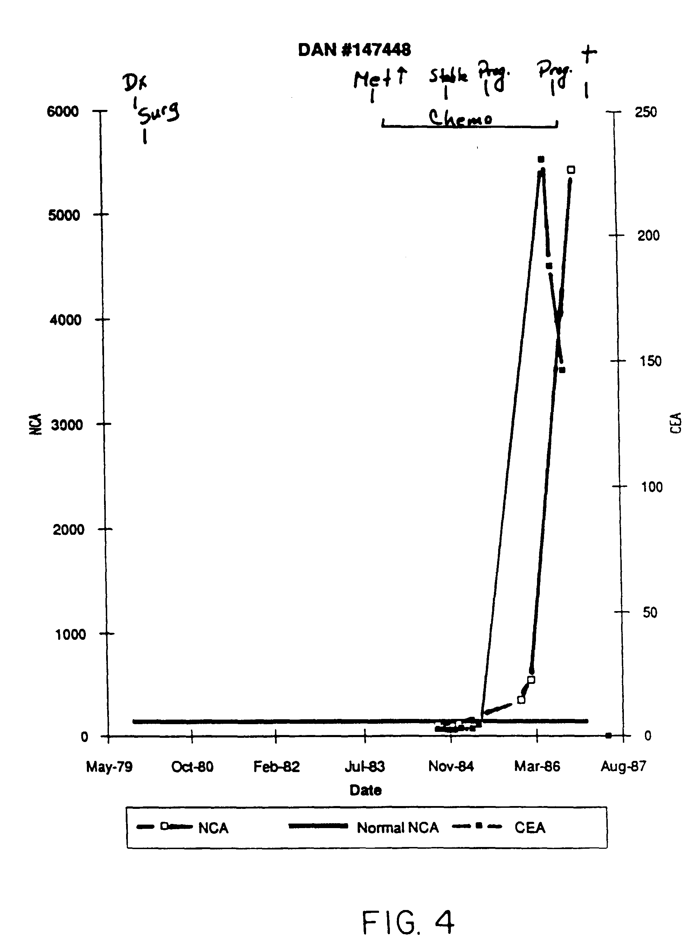

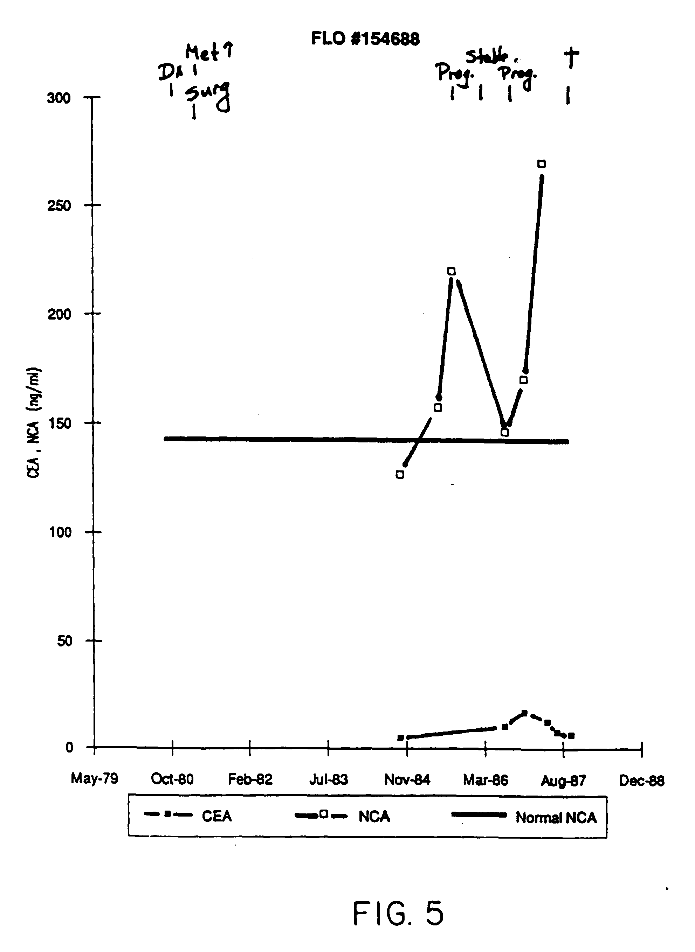

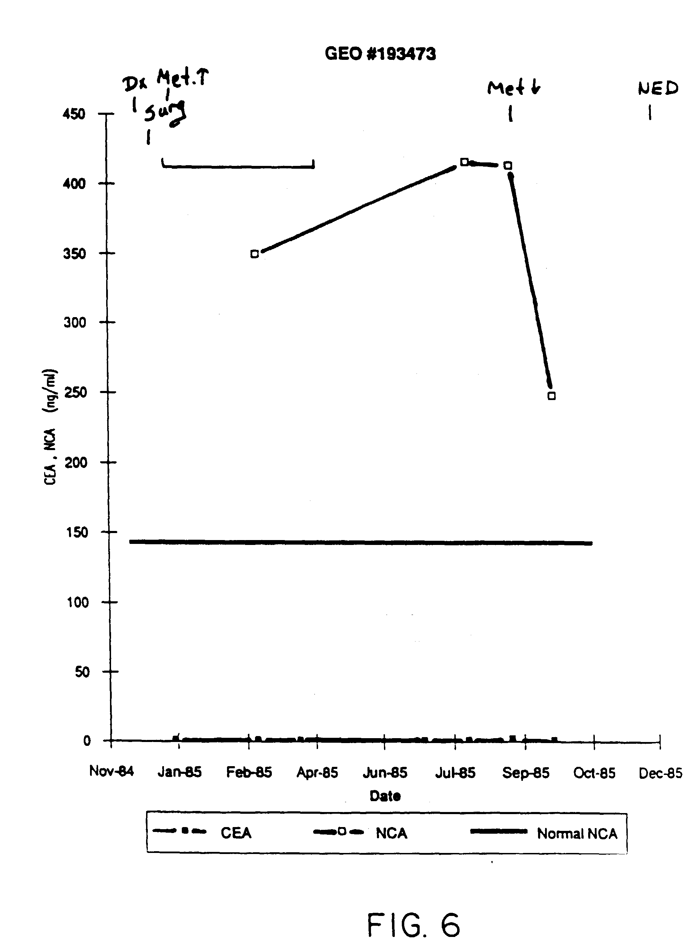

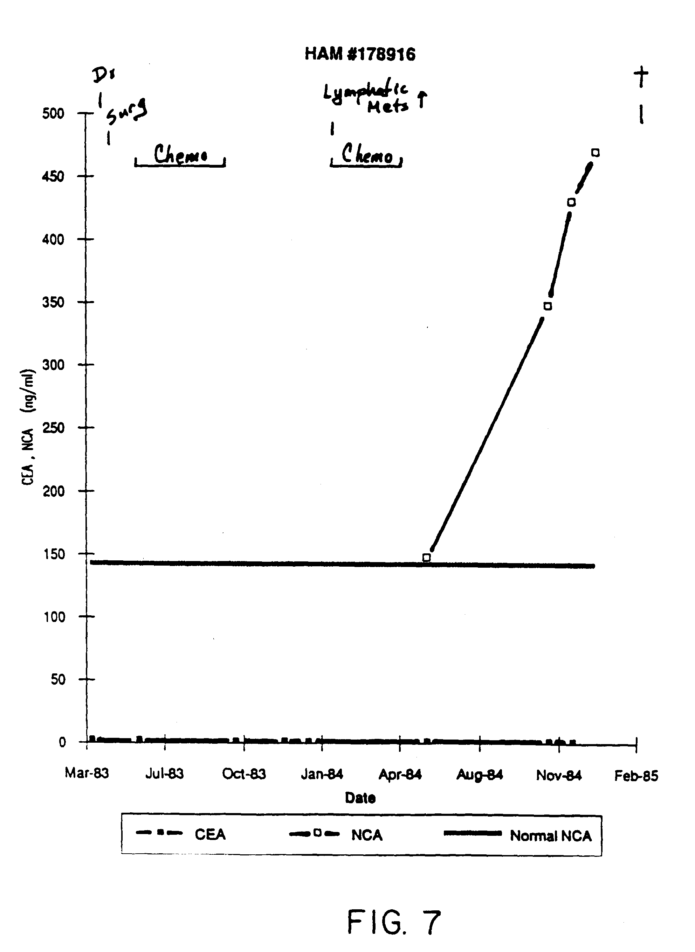

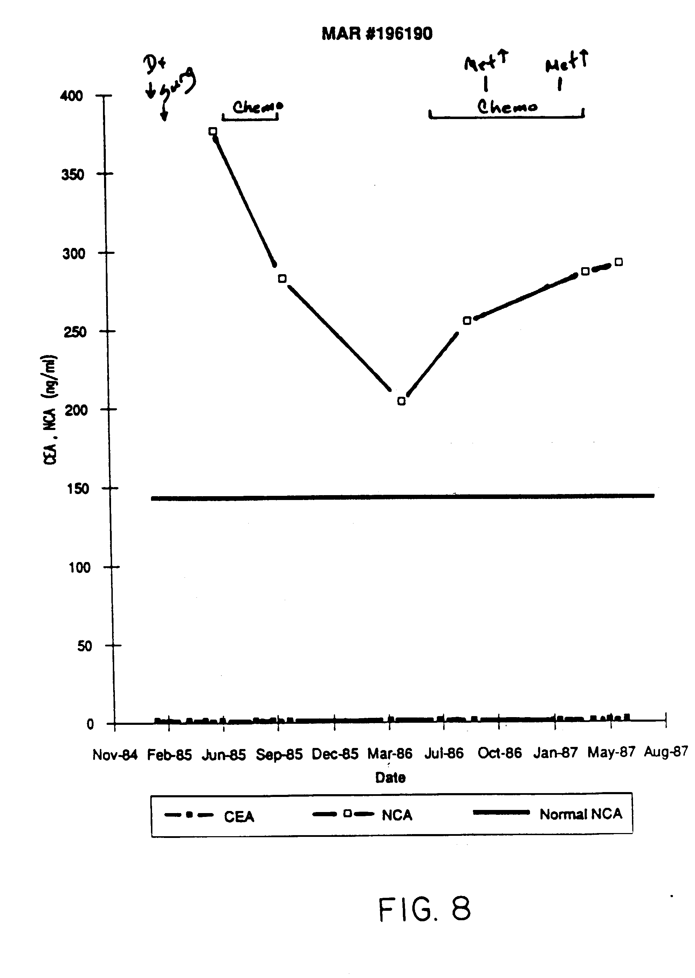

Changes in the serum levels of NCA-BT were found to correlate with the stage of disease in a significant number of patients diagnosed and under treatment for breast cancer. Serial serum samples were obtained from clinically confirmed breast cancer patients. These serial samples had a mean value of 209 ng/ml of NCA-BT. Six examples of the correlation of serum CEA and NCA-BT levels and the clinical status of these patients are shown in Figs. 3-8.

The graphs each show:
(a) the levels of NCA-BT as measured by the above-described sandwich immunoassay method,
(b) the levels of serum CEA that had been measured in a clinical laboratory and shown on the patient's chart,
(c) the 95th percentile cut-off value for normal NCA-BT (i.e., 143 ng/ml), and
(d) across the top of the graph, a summary of the disease progression using the following abbreviations:
   - "Dx" =: clinical diagnosis,
   - "Surg" =: surgical procedure to remove the cancer,
   - "Chemo" =: period during which chemotherapy was administered,
   - "Stable" =: an assessment by the attending physical that the patient's breast cancer has stabilized,
   - "Met↑" =: an assessment by the attending physician that metastasis had occurred or that the breast cancer was otherwise progressing,
   - "Prog" =: an assessment by the attending physician that the breast cancer was progressing,
   - "+" =: death of the patient,
   - "Met↓" =: an assessment by the attending physician that metastasis had been arrested, and
   - "NED" =: an assessment by the attending physician that there was no longer any evidence of disease.

In these examples, not only does serum NCA-BT show correlation with the clinical regression, stability, and progression of the disease, but also such correlation is more significant than the correlation with serum CEA levels (a commonly used serum marker for monitoring breast cancer).

The high incidence of elevated NCA-BT levels in breast cancer sera, and the significant correlation of clinical status with changes in the NCA-BT levels shows that the present method is useful as a means for monitoring the progression of breast cancer in patients.

## Claims

1. A method for monitoring the progression of breast cancer in a patient, characterized in that a series of specific immunoassays are performed over time to determine changes in the level of NCA-BT in blood samples obtained from such patient, whereby increases in blood NCA-BT levels indicate a deteriorating condition while decreases in blood NCA-BT levels indicate an improving condition.

2. The method of claim 1 wherein the immunoassays performed are sandwich immunoassays in which at least one of the antibody reagents is specific for NCA-BT with no substantial reactivity for CEA, NCA-CML, or TM-CEA.

3. The method of claim 2 wherein said NCA-BT specific antibody reagent is a monoclonal antibody reagent.

4. The method of claim 1 wherein said blood samples are serum samples.

5. A sandwich immunoassay test kit for use in the determination of NCA-BT in a blood sample, comprising first and second antibody reagents, characterized in that said first antibody reagent is a NCA-BT specific antibody reagent with no substantial reactivity for CEA, NCA-CML or TM-CEA and said second antibody reagent is capable of binding specifically or nonspecifically with NCA-BT, preferably wherein one of said antibody reagents if labelled with an enzyme.

6. The test kit of claim 5 wherein said first antibody reagent is monoclonal.

7. The test kit of claim 6 wherein said second antibody reagent is also monoclonal.

## Patentansprüche

1. Verfahren zur Aufzeichnung des Verlaufs von Brustkrebs in einem Patienten, **gekennzeichnet dadurch, daß** eine Serie von spezifischen Immunoassays über einen Zeitraum zur Bestimmung von Änderungen des NCA-BT-Wertes in von diesen Patienten erhaltenen Blutproben durchgeführt wird, wobei Erhöhungen der Blut-NCA-BT-Werte eine Verschlechterung des Zustandes, und Abnahmen der Blut-NCA-BT-Werte eine Verbesserung des Zustandes anzeigen.

2. Verfahren gemäß Anspruch 1, worin die durchgeführten Immonoassays Sandwich-Immunoassays sind, bei denen mindestens eines der Antikörperreagentien spezifisch für NCA-BT ohne wesentliche Reaktivität für CEA, NCA-CML oder TM-CEA ist.

3. Verfahren gemäß Anspruch 2, worin das spezifische NCA-BT Antikörperreagenz ein monoklonales Antikörperreagenz darstellt.

4. Verfahren gemäß Anspruch 1, worin die Blutproben Serumproben sind.

5. Sandwich-Immunoassay-Testkit zur Verwendung bei der Bestimmung von NCA-BT in einer Blutprobe, umfassend erste und zweite Antikörperreagentien, **gekennzeichnet dadurch, daß** das erste Antikörperreagenz ein spezifisches NCA-BT Antikörperreagenz ohne wesentliche Reaktivität für CEA, NCA-CML oder TM-CEA und das zweite Antikörperreagenz spezifisch oder unspezifisch an NCA-BT binden kann, worin bevorzugt einer der Antikörperreagenzien mit einem Enzym markiert ist.

6. Testkit gemäß Anspruch 5, worin das erste Antikörperreagenz monoklonal ist.

7. Testkit gemäß Anspruch 6, worin das zweite Antikörperreagenz auch monoklonal ist.

## Revendications

1. Procédé de contrôle de l'évolution du cancer du sein chez une patiente, caractérisé en ce qu'une série d'essais immunologiques spécifiques sont effectués dans le temps pour déterminer les changements dans les taux de NCA-BT dans les échantillons de sang obtenus auprès d'une telle patiente, les augmentations dans les taux sanguins de NCA-BT indiquant une condition de détérioration alors que les diminutions dans les taux sanguins de NCA-BT indiquent une condition d'amélioration.

2. Procédé selon la revendication 1, dans lequel les essais immunologiques effectués sont des essais immunologiques en sandwich dans lesquels au moins un des anticorps est spécifique de NCA-BT sans aucune réactivité substantielle pour les CEA, NCA-CML ou TM-CEA.

3. Procédé selon la revendication 2, dans lequel ledit anticorps spécifique de NCA-BT est un anticorps monoclonal.

4. Procédé selon la revendication 1, dans lequel lesdits échantillons sanguins sont des échantillons de sérum.

5. Coffret d'analyse pour essai immunologique en sandwich pour l'utilisation dans la détermination de NCA-BT dans un échantillon de sang, comprenant le premier et le second anticorps, caractérisé en ce que ledit premier anticorps est un anticorps spécifique de NCA-BT sans aucune réactivité substantielle avec les CEA, NCA-CML ou TM-CEA et ledit second anticorps est capable de liaison spécifique ou non spécifique avec le NCA-BT, de préférence dans lequel un desdits anticorps est marqué avec une enzyme.

6. Coffret d'analyse selon la revendication 5, dans lequel ledit premier anticorps est monoclonal.

7. Coffret d'analyse selon la revendication 6, dans lequel ledit second anticorps est aussi monoclonal.
